# EUROPÄISCHE PATENTSCHRIFT

(11) **EP 1 713 922 B1**
(45) Veröffentlichungstag und Bekanntmachung des Hinweises auf die Patenterteilung: **04.01.2012**
(21) Anmeldenummer: 05715291.0
(22) Anmeldetag: 10.02.2005
(51) Int. Cl.: C12P 5/00, C12R 1/645

(54) **Verfahren zur Herstellung von aromaaktiven Terpenen**
Process for producing flavor-active terpenes
Procédé de production de terpènes à effet aromatique

(30) Priorität: 11.02.2004 DE 102004006825
(43) Veröffentlichungstag der Anmeldung: 25.10.2006
(73) Patentinhaber: Maxens GmbH, 83308 Trostberg (DE)
(72) Erfinder: MÜLLER, Martin, 59494 Soest (DE); DIRLAM, Kerstin, 76437 Rastatt (DE); WENK, Hans, Henning, 85354 Freising (DE); BERGER, Ralf, G., 30455 Hannover (DE); KRINGS, Ulrich, 31628 Landesbergen (DE); KASPERA, Rüdiger, 26125 Oldenburg (DE)
(74) Vertreter: Metten, Karl-Heinz
(86) Internationale Anmeldenummer: PCT/EP2005/001346
(87) Internationale Veröffentlichungsnummer: WO 2005/078110

(56) Entgegenhaltungen:
- EP-A- 1 424 071
- ONKEN J ET AL: "Effects of R-(+)-limonene on submerged cultures of the terpene transforming basidiomycete Pleurotus sapidus" JOURNAL OF BIOTECHNOLOGY, ELSEVIER SCIENCE PUBLISHERS, AMSTERDAM, NL, Bd. 69, Nr. 2-3, 15. April 1999 (1999-04-15), Seiten 163-168, XP004168124 ISSN: 0168-1656
- CROAN SUKI C: "Lyophilization of hypha-forming tropical wood-inhabiting Basidiomycotina" MYCOLOGIA, Bd. 92, Nr. 4, Juli 2000 (2000-07), Seiten 810-817, XP008049845 ISSN: 0027-5514
- SUNDARI S KRISHNA ET AL: "Freeze-drying vegetative mycelium of Laccaria fraterna and its subsequent regeneration" BIOTECHNOLOGY TECHNIQUES, Bd. 13, Nr. 7, Juli 1999 (1999-07), Seiten 491-495, XP008049838 ISSN: 0951-208X
- TAUBERT J ET AL: "A comparative study on the disintegration of filamentous fungi" JOURNAL OF MICROBIOLOGICAL METHODS, Bd. 42, Nr. 3, November 2000 (2000-11), Seiten 225-232, XP002335974 ISSN: 0167-7012
- KASPERA RÜDIGER ET AL: "Bioconversion of (+)-valencene in submerged cultures of the ascomycete Chaetomium globosum." APPLIED MICROBIOLOGY AND BIOTECHNOLOGY. JUN 2005, Bd. 67, Nr. 4, Juni 2005 (2005-06), Seiten 477-483, XP002335975 ISSN: 0175-7598

## Beschreibung

Gegenstand der vorliegenden Erfindung ist ein Verfahren zur Herstellung von aromaaktiven Terpenen mit Hilfe einer selektiven Biotransformation.

Aromen und Riechstoffe spielen in der heutigen Gesellschaft eine große Rolle. Geruchsaktive Stoffe werden in einer Vielzahl von Produkten des täglichen Lebens, wie Parfümen, Kosmetika, Lebensmitteln, Pharmaka und Haushaltsprodukten, verwendet Beispielsweise werden 15 % aller auf dem Markt befindlichen Lebensmittel durch Zusätze aromatisiert. Die Gewinnung von Aromastoffen mittels Extraktion oder Destillation erfolgt auch heute noch aus verschiedensten Pflanzenteilen (Früchte, Blüten, Samen, Wurzeln u.v.m). Extrakte oder auch isolierte Verbindungen kommen als hochwertige Produkte (z.B. in der Parfümindustrie als "essence absolue") in den Handel.

Der weiterhin zunehmend große Bedarf an Aromastoffen kann nicht mehr allein über natürliche Extrakte gedeckt werden, weshalb die Herstellung von Aromastoffen zu ca. 80 % durch chemische Synthesen erfolgt. Die Synthesestufen sind oft aufwendig, wenig spezifisch und so können die Produkte gemäß der Aromenverordnung (i.d.F. vom 18.06.2001) lebensmittelrechtlich lediglich als "naturidentisch", aber nicht als "natürlich" deklariert werden. Laut Aromenverordnung können natürliche Aromen "...durch enzymatische oder mikrobiologische Verfahren aus Ausgangsstoffen pflanzlicher oder tierischer Herkunft..." hergestellt werden. Deshalb ist die Entwicklung biotechnologischer Verfahren zur Herstellung von natürlichen Aromastoffen eine sinnvolle Alternative. Die Herstellung von Aromen mittels Biotransformationen, d.h. der biokatalytischen Umwandlung von Ausgangskomponenten zu Syntheseprodukten durch z.B. mikrobielle Metabolisierung, spricht den erzeugten Aromastoffen entsprechend weiterhin das Prädikat "natürlich" zu, womit sie vor allem beim Konsumenten eine entscheidend höhere Akzeptanz gewinnen.

Eine wichtige Gruppe der Aromastoffe bilden die Terpenkohlenwasserstoffe und ihre Oxidationsprodukte, die Terpenoide. Als weit verbreitete Naturstoffe sind ihre sensorischen oder pharmakologischen Wirkungen schon seit langer Zeit bekannt. Mono- und Sesquiterpene dienen als Produkte des Sekundärstoffwechsels in der Pflanzen- und Tierwelt als Lockstoffe oder aufgrund ihrer toxischen Wirkung als Fraßschutz. Sie wirken ebenfalls als Signalstoffe und Phytohormone. So dienen z.B. mit der pflanzlichen Nahrung aufgenommene und metabolisierte Terpene den Insekten als Soziohormone oder Kommunikationspheromone. Olfaktorisch aktive Terpene zeichnen sich häufig durch außerordentlich niedrige Geruchsschwellen aus und bilden als sogenannte "character impact compounds" den aromabildenden Inhaltsstoff einer bestimmten Aromanote, z.B. Rosenoxid für Geranienduft mit einer Geruchsschwelle von 0,5 µg kg⁻¹ als typisches Orangenaroma.

Als Ausgangsverbindungen zur Herstellung von Terpenoiden bieten sich ihre natürlichen Vorstufen an. Diese weniger interessanten Mono- wie auch Sesquiterpenkohlenwasserstoffe werden von den hochwertigen Terpenoiden abgetrennt und fallen als "Abfall" in Tonnenmaßstäben an. So wird das Monoterpen R-(+)-Limonen als Abfallstoff aus der Orangenölverarbeitung in Mengen von mehr als 100.000 t pro Jahr produziert und preiswert gehandelt. Es kommt als Hauptkomponente des Orangenschalenöls mit einem Gehalt von mehr als 90 % vor und fällt bei der Rektifizierung an. Aufgrund ihrer nahezu unbegrenzten Verfügbarkeit und ihrer strukturellen Ähnlichkeit bilden die Terpenkohlenwasserstoffe ideale Grundstoffe zur Herstellung der korrespondierenden Oxidationsprodukte durch chemische oder biokatalytische Synthesen.

Für eine Terpenbiotransformation kann auf eine nahezu unbegrenzte Anzahl an Biokatalysatoren wie z.B. Bakterien, Hefen, Pilze und Pflanzenzellen zurückgegriffen werden, wobei sich Pilze als besonders aktive Biokatalysatoren herausgestellt haben. Nach heutigem Kenntnisstand sind mehr als 100.000 Arten aus dem Reich der Mycobionta (Pilze) bekannt, von denen einige Organismen der Wirtschaft Zugang zur Produktion einer Reihe wichtiger Verbindungen, wie z.B. Antibiotika, Vitamine, organische Säuren, verschafft haben. In der Biotechnologie unterscheidet man hierbei zwischen der *de novo* Herstellung, also der direkten Ausscheidung als Stoffwechselprodukt durch vitale Zellsysteme und der Biotransformation, mit der strokturähnliche Vorstufen (Precursoren) durch gezielte funktionelle Reaktionen in die gewünschten Zielprodukte umgewandelt werden. Neben der direkten Produktion ist auch eine Vielzahl von Mikroorganismen in der Lage, xenobiotische und makromolekulare Substrate abzubauen und zu metabolisieren.

Insbesondere höhere Pilze, wie Basidiomyceten, besitzen durch ihr natürliches Habitat eine Vielzahl an oxidativ wirkenden Enzymen für den Holzabbau (z.B. Laccasen, Peroxidasen). Die zurzeit bekannten ca. 30.000 Arten der Basidiomyceten bieten sich daher besonders für oxidative Biotransformationen von Terpenkohlenwasserstoffen an. Bspw. kann der von Pflanzen ausgeschiedene Abwehrstoff α-Pinen durch eine mikrobielle Oxidation detoxifiziert werden. Der Vorteil von Pilzen gegenüber niederen Organismen, wie Bakterien, ist die wesentlich vielfältigere Ausstattung an Redoxenzymen mit hohem Oxidationspotential, was sie insbesondere zur Oxidation xenobiotischer Substanzen befähigt.

Das Dokument ONKEN J. & BERGER, R.G.: "Effects of R-(+)-limonene on submerged cultures of the terpene transforming basidiomycete Pleurotus sapidus" (JOURNAL OF BIOTECHNOLOGY, Bd. 69, Nr. 2-3, 15. April 1999, Seiten 163-168) offenbart ein Verfahren zur Herstellung von Carvon aus Limonen unter Verwendung einer submersen *Pleurotus sapidus Kultur*. Dabei wird kein lyophilisiertes Mycel eingesetzt.

Das Dokument CROAN, S.C.: "Lyophilization of hypha-forming tropical wood-inhabiting Basidiomycotina" (MYCOLOGIA, Bd. 92, Nr. 4, Juli 2000, Seiten 810-817) offenbart, dass die Mycelien verschiedener Basidomycotina auch nach der Lyophilisierung überlebensfähig sind, einige sogar gesteigerte Wachstumsraten aufweisen.

Das Dokument SUNDARI, S.K. & ADHOLEYA, A.: "Freeze-drying vegetative mycelium of Laccaria fratema and its subsequent regeneration" (BIOTECHNOLOGY TECHNIQUES, Bd. 13, Nr. 7, Juli 1999, Seiten 491-495) offenbart, dass das Mycel von *Laccaria fratema* auch nach der Lyophilisierung überlebensfähig ist und keine veränderte Wachstumsrate aufweist.

Aufgrund ihrer strukturellen Komplexität stellt die chemische Totalsynthese von Sesquiterpenoiden die Aromaindustrie allerdings vor außergewöhnliche Schwierigkeiten. Am einfachsten kann sie durch die Funktionalisierung von natürlichen Vorstufen durchgeführt werden, wobei sich chemische Synthesestufen aber als wenig selektiv herausgestellt haben.

Aus den bekannten Problemen des Standes der Technik hat sich für die vorliegende Erfindung deshalb die Aufgabe gestellt, ein Verfahren zur Herstellung von aromaaktiven Terpenen aus Terpenkohlenwasserstoffen zur Verfügung zu stellen, das im Rahmen einer selektiven Biotransformation und mit Hilfe von Mikroorganismen der Klasse Ascomycetes, Basidiomycetes und Deuteromycetes durchgeführt wird. Dabei stand die Bereitstellung eines Verfahrens im Vordergrund, welches auf einfache und wirtschaftliche Weise durchzuführen ist, dabei die selektiven Eigenschaften von enzymatischen Prozessen nutzt und ausgehend von leicht zugänglichen und kostengünstigen Ausgangsstoffen zu hochwertigen Produkten mit ausgeprägter Reinheit führt, die insbesondere für lebensmitteltechnische Anwendungen geeignet sind.

Gelöst wurde diese Aufgabe mit Hilfe eines entsprechenden Verfahrens, bei dem ein lyophilisiertes Mycel eingesetzt wird, welches zuerst rehydratisiert und dann mit dem Substrat versetzt wird.

Überraschend hat sich dabei herausgestellt, dass durch den Verfahrensschritt der Perforierung der Mycel-Zellen durch Lyophilisierungsmaßnahmen Enzymsysteme in Ganzzellkulturen genutzt werden können, wobei das Kulturmedium mit keinen zusätzlichen Aktivatoren versetzt werden muss. Gemäß Aufgabenstellung werden mit diesem Verfahren nicht nur die gewünschten aromaaktiven Terpene in hervorragenden Qualitäten erhalten, sondern es ist auch möglich, durch die Auswahl geeigneter Mikroorganismen die gewünschten Verbindungen enantio-, stereo- bzw. regioselektiv herzustellen und durch verfahrenstechnisch einfache Maßnahmen aus dem Reaktionsmedium zu gewinnen. Insbesondere war bislang die enantioselektive Herstellung von Monoterpeoiden durch Biotransformation aufgrund des Mangels an geeigneten Organismen und Enzymen nur sehr beschränkt möglich. Aufgrund der bisherigen Schwierigkeiten, wie sie aus chemischen Verfahren bekannt sind, aber auch aus Verfahrensvarianten der Biotransformation, waren die Vorteile des erfindungsgemäßen Verfahrens in dieser Ausprägung nicht zu erwarten.

Wie bereits angedeutet, ist ein erfindungswesentlicher Vorteil des vorliegenden Verfahrens darin zu sehen, dass ein lyophilisiertes Mycel eingesetzt wird. Um die Vorteile dieses Verfahrensmerkmals noch besser nutzen zu können, sieht die vorliegende Erfindung vor, dass ein Mycel eingesetzt wird, dessen Zellen zusätzlich durch Ultraschallbehandlung und/oder eine Extrusion permeiert wurden.

Die verwendeten zellulären Biokatalysatoren können somit vor ihrem Einsatz in der eigentlichen Transformationsreaktion so vorbehandelt werden, dass die Ausgangsverbindungen zunächst die Zellwand durchdringen und anschließend in die Zellmembranen diffundieren können. Die Nachteile der Zellmembran als osmotische Barriere werden damit überwunden und die damit üblicherweise verbundene und bislang bekannte Hemmung der Biotransformation, wie sie bspw. in Form einer Verlangsamung des Influx von Substraten und des Eflux von Produkten stattfindet, kann reduziert oder gänzlich vermieden werden. Durch die Perforierungsmaßnahmen kann der Austausch an Substraten deutlich beschleunigt werden, da hauptsächlich durch die Lyophilisierung eine Störung der Membranintegrität bewirkt wird, wobei allerdings die darin enthaltenen Enzymsysteme intakt bleiben, gleichzeitig aber leichter zugänglich werden.

Als besonders günstig hat es sich erwiesen, wenn das vorgeschlagene Verfahren in submerser Kultur durchgeführt wird. Ein weiterer Vorteil des erfindungsgemäßen Verfahrens ist darin zu sehen, dass die Biotransformation enantioselektiv, stereoselektiv und/oder regioselektiv durchgeführt werden kann.

Eine bedeutende Rolle für das Gelingen des erfindungsgemäßen Verfahrens kommt der Auswahl geeigneter Mikroorganismen zu. In diesem Zusammenhang berücksichtigt die vorliegende Erfindung eine Variante, bei der als Biokatalysatoren Vertreter von Fusarium, Pleurotus, Penicillium und Chaetomium eingesetzt werden. Als besonders geeignet haben sich *Fusarium proliferatus*, *Pleurotus sapidus*, *Penicillium citrinum* und *Chaetomium globosum* erwiesen.

Im Hinblick auf die zu gewinnenden aromaselektiven Terpene werden von der vorliegenden Erfindung Mono- und Sesquiterpene als Ausgangsterpenkohlenwasserstoffe bevorzugt, wobei Limonen und insbesondere R-(+)-Limonen oder S-(-)-Limonen sowie Pinen, Valencen, Farnesen, Thymol und Dimethylallylalkohol als besonders geeignet anzusehen sind.

In bestimmten Fällen kann es günstig sein, im lyophilisierten Mycel vor der eigentlichen Biotransformation eine Enzyminduktion durchzuführen, wofür sich die Zugabe von Substrat als geeignet erwiesen hat. Üblicherweise werden die Lyophilisate des Mycels nach ihrer Rehydratisierung in einem Puffer mit einer bestimmten Menge an Substrat versetzt, wodurch sich die Pilzkultur adaptiert und eine Induktion von Enzymen, die zur Terpenoxidation geeignet sind, erreicht wird. Die eigentliche Zugabe des Ausgangsterpenkohlenwasserstoffs erfolgt dabei nach wenigen Stunden bis zwei Tagen.

Die vorliegende Erfindung sieht als weitere bevorzugte Variante vor, die Biotransformation in einem zweiphasigen System aus Wasser und einer organischen Phase durchzuführen, wobei sich insbesondere n-Decan als geeignete Phase erwiesen hat. Besonders bevorzugt wird die Biotransformation ohne Zugabe von Co-Solventien durchgeführt, was die vorliegende Erfindung ebenfalls berücksichtig.

Ein weiterer Vorteil des erfindungsgemäßen Verfahrens besteht darin, dass die Biotransformationsreaktion in einem Medium durchgeführt werden kann, das eine verringerte Menge M der sonst üblichen Kohlenstoffquelle, wie bspw. Glucose, enthält, wodurch eine höhere Biotransformation des angegebenen Substrats erfolgt. M ist vorzugsweise < 50 gL⁻¹, stärker bevorzugt < 25 gL⁻¹ und am stärksten bevorzugt < 10 gL⁻¹.

Üblicherweise werden Biotransformationsreaktionen in wässrigen Systemen durchgeführt, wobei die Verwendung organischer Lösemittel die Verfügbarkeit lipophiler Substrate erhöht, wenn das Verteilungsgleichgewicht von Edukt und Substraten/Produkten im wässrigen Medium nachteilig ist. Wie bereits beschrieben, wurde ein geeignetes Lösemittel in Form eines Zweiphasensystems ermittelt, wobei n-Decan die organische Phase darstellt. Wird n-Decan demgegenüber als Co-Solvents eingesetzt, kann es in Abhängigkeit vom eingesetzten Mycel zu einer Aktivitätsinhibierung bei den Enzymen kommen, weshalb die vorliegende Erfindung auch empfiehlt, auf Co-Solventien zu verzichten.

Im Hinblick auf die Endprodukte sieht das erfindungsgemäße Verfahren vor, diese aus zellulären Komponenten oder Zellfraktionen des Mycels zu isolieren. Üblicherweise reichern sich lipophile Substanzen zu über mehr als 90 % im Mycel und hier insbesondere in Zellwand- und Membranfraktionen an. Ein verschwindend geringer Teil von ca. nur 5 % wird im wässrigen Medium gefunden.

Da es für eine erfolgreiche Biotransformation auch einer optimalen Sauerstoffzufuhr bedarf, empfiehlt es sich, das vorgeschlagene Verfahren in entsprechenden Vorrichtungen, wie bspw. Rührkessel-, Oberflächen- und Festbettreaktoren durchzuführen, welche die vorliegende Erfindung insbesondere empfiehlt. Der Stoffwechselweg der Terpene im jeweiligen Mikroorganismus spielt bei zahlreichen Biotransformationen eine wichtige Rolle. Dabei ist bekannt, dass für einige Mikroorganismen eine Co-Oxidation der Terpensubstrate eine Rolle spielt, ohne dass eine Weiteroxidation und Metabolisierung stattfindet. Insbesondere wenn nährstoffreiche Medien verwendet werden, erscheint eine Metabolisierung von Terpenen als C-Quelle nicht notwendig. Andererseits können Terpenkohlenwasserstoffe als alleinige C-Quelle dienen und über eine β-Oxidation metabolisiert werden. Insbesondere im Hinblick auf die im Kulturmedium anwesenden Kohlenstoffquellen hat es sich für das vorliegende Verfahren als vorteilhaft gezeigt, dass ein Glucose-Gehalt G mit G ≤ 0,5% zur Anzucht transformationsaktiver Biomassen ausreicht. Ebenfalls bestätigt wurde, dass die organischen Hauptkomponenten des Kulturmediums, nämlich Kohlenstoff und Stickstoff, einen entscheidenden Einfluss auf die Transformationsausbeute haben. Dabei hat sich wieder als vorteilhaft erwiesen, wenn ein kohlenstoffarmes Medium, wie bereits angesprochen, verwendet wird, um so den Anteil an Oxidationsprodukten zu erhöhen, wobei es gelingt, eine weitergehende Mineralisierung des Zielproduktes zu unterbinden.

Insbesondere den ebenfalls bereits angesprochenen Schüttelvorrichtungen kommt eine weitere wichtige Rolle dahingehend zu, als Sauerstoff ein essentielles Co-Substrat der Oxidation von Terpenkohlenwasserstoffen ist und weshalb vor allem obligat aerobe Pilze, wie Ascomyceten, zur Aufrechterhaltung lebensnotwendiger Prozesse und für eine optimale Biomasseproduktion ausreichende Sauerstoffmengen benötigen. Somit ist die Verfügbarkeit von Sauerstoff während einer Kultivierung ausreichend zu gewährleisten, was mit den angesprochenen Rührkessel-, Oberflächen- oder Festbettreaktoren gelingt. Hinzu kommt eine zusätzliche Oberflächenvergrößerung der Schüttelkultur, womit ein erhöhter Gasaustausch und verbesserte Masseniransfer-Koeffizienten einhergehen.

Das vorliegende Verfahren hat sich insbesondere als geeignet erwiesen, als Endprodukte terpenoide Alkohole, Epoxide, Aldehyde, Ketone, Mehrfach-Alkohole, Carbonyle und Carbonylalkohole zu erhalten. Besonders bevorzugt in diesem Zusammenhang werden Piperiton, Isopiperiton, Isopiperitenol, Isopiperitenon, Perillaaldehyd, Carvon, Carveol, Linalool, Linalooloxid, Terpineol sowie Nootkatol und Nootkaton angesehen.

Schließlich berücksichtigt die vorliegende Erfindung auch noch Verfahrensvarianten, mit denen gezielt aromaaktive Terpene hergestellt werden können. So wird insbesondere empfohlen, erst enantioselektiv R-(+)-Limonen zu cis-(+)Carveol und S-(-)-Limonen zum trans-(-)-Carveol zu biotransformieren, wofür sich insbesondere spezielle Fusarium-Arten als Biokatalysatoren als geeignet erwiesen haben. Anschließend kann das so erhaltene trans-(-)-Carveol zum R-(-)-Carvon umgesetzt werden, wobei dann *Pleurotus spec.*-Stämme verwendet werden sollten.

Die vorliegende Erfindung umfasst ebenfalls die Biotransformation bicyclischer Sesquiterpene zu β-Nootkatol und anschließend zu Nootkaton, wofür Chaetomium-Spezies empfohlen werden.

Das beschriebene Verfahren ermöglicht die Veredelung bestimmter Terpenkohlenwasserstoffe, wie bspw. Limonen, Valencen und Farnesen zu hochwertigen aromaaktiven Verbindungen, wie Carvon, Nootkaton und 7-Hydroxy-Farnesen durch eine mikrobielle Biotransformation. Dabei ist es möglich, die gewünschten aromaaktiven Terpene durch Auswahl geeigneter Pilzkulturen und insbesondere die Anwendung der daraus gewonnenen lyophilisierten Mycele in wirtschaftlich vorteilhafter Weise in größeren Mengen und sehr guter Qualität zu erhalten, wobei die biokatalytische Reaktionsführung insgesamt einfach in submerser Kultur in geeigneten Vorrichtungen durchgeführt werden kann.

Die nachfolgenden Beispiele veranschaulichen die Vorteile des beanspruchten Verfahrens.

### Beispiele

Für die nachfolgenden Beispiele wurden als Biokatalysatoren Mycelien eingesetzt, die in Submers-Kulturen bei 24 °C und 150 Upm angezüchtet wurden.

Nach 3 bis 7 Tagen Wachstumszeit wurden von diesen Vorkulturen 10 mL homogenisiertes Medium in 200 mL SNLH-Medium überfährt und bei 24 °C und 150 Upm kultiviert. Zur Adaptation der Kultur wurden anschließend 20 µL des jeweiligen Terpens nach drei bis fünf Tagen Wachstunnszeit zur Kultur gegeben. Die Zellmasse wurde dann durch Zentrifugation (2000 g, 10 min.) abgetrennt, mit 0,9 %iger Natriumchloridlösung gewaschen und unter flüssigem Stickstoff schockgefroren. Die Gefriertrocknung bzw. Lyophilisierung (Anlage Finaqua Lyovac GT2) wurde bei Raumtemperatur und bei 2 x 10⁻⁵ bar für ein bis vier Tage (je nach Kultur) durchgeführt.

### Transformatzonsbedingungen:

Zur Rehydratisierung der gefriergetrockneten Zellmasse wurde zerkleinertes Lyophilisat im Transformationsmedium (z. B. MOPS-Puffer, 4-[N-Morpholino] butansulfonsäure, Hefe-Nähmedium nach Sprecher und Hansen [1982]) 1 bis 24 h inkubiert. Die Zugabe des Terpenkohlenwasserstoffs (1 bis 300 mM) erfolgte direkt oder unter Verwendung von Lösemitteln. Die Terpenoidbildung wurde durch kontinuierliche Probenahme von Aliquots bestimmt. Die Terpenoide wurden durch Lösemittel-Extraktion gewonnen. Die Identifikation der Verbindungen erfolgte mittels GC-MS über authentische Standards, die Quantifizierung über GC-FID und die verwendeten internen Standards.

### Beispiel 1:

Zur Transformation von Limonen wurden vom *Pleurotus* sapidus-Mycel 50 mg in 1,5 mL MOPS-Puffer (0,1 M; pH 7,0) gegeben und die getrocknete Zellmasse für eine Stunde bei 200 Upm und 24 °C rehydratisiert. Zur Carvon-Erzeugung wurden 41 mM Limonen direkt zu der rehydratisierten Kultur appliziert. Die Umsetzung erfolgte für 24 h bei 150 Upm und 24 °C. Proben wurden nach Zugabe des inneren Standards (z.B. Campher für Limonentransformation) mit 2 mL azeotropem Pentan/Ether-Gemisch extrahiert, zentrifugiert und über Nacht mit Na₂SO₄ getrocknet.

## Patentansprüche

1. Verfahren zur Herstellung von aromaaktiven Terpenen aus Terpenkohlenwasserstoffen mittels einer selektiven Biotransformation und mit Hilfe von Mikroorganismen der Klassen Ascomycetes, Basidiomycetes und Deuteromycetes, **dadurch gekennzeichnet, dass** ein lyophilisiertes Mycel eingesetzt wird, das zuerst rehydratisiert und dann mit dem Substrat versetzt wird.

2. Verfahren nach Anspruch 1, **dadurch gekennzeichnet, dass** die Mycel-Zellen zusätzlich durch Ultraschallbehandlung und/oder Extrusion permeiert werden.

3. Verfahren nach einem der Ansprüche 1 oder 2, **dadurch gekennzeichnet, dass** die Biotransformation in submerser Kultur vorgenommen wird.

4. Verfahren nach einem der Ansprüche 1 bis 3, **dadurch gekennzeichnet, dass** die Biotransformation enantio-, stereo- und/oder regioselektiv durchgeführt wird.

5. Verfahren nach einem der Ansprüche 1 bis 4, **dadurch gekennzeichnet, dass** als Mikroorganismen Vertreter von Fusarium, Pleurotus, Penicillium und Chaetomium eingesetzt werden.

6. Verfahren nach einem der Ansprüche 1 bis 5, **dadurch gekennzeichnet, dass** als Mikroorganismen Fusarium proliferatus, Pleurotus sapidus, Penicillium citrinum und Chaetomium globosum eingesetzt werden.

7. Verfahren nach einem der Ansprüche 1 bis 6, **dadurch gekennzeichnet, dass** als Terpenkohlenwasserstoffe Mono- und Sesquiterpene verwendet werden.

8. Verfahren nach einem der Ansprüche 1 bis 7, **dadurch gekennzeichnet, dass** als Terpenkohlenwasserstoffe Limonen, Pinen, Valencen, Farnesen, Thymol und Dimethylallylalkohol verwendet werden.

9. Verfahren nach einem der Ansprüche 1 bis 8, **dadurch gekennzeichnet, dass** als Terpenkohlenwasserstoffe R-(+)-Limonen oder S-(-)-Limonen verwendet werden.

10. Verfahren nach einem der Ansprüche 1 bis 9, **dadurch gekennzeichnet, dass** im lyophilisierten Mycel vor der Biotransformation durch Zugabe von Substrat eine Enzyminduktion durchgeführt wird.

11. Verfahren nach einem der Anspniche 1 bis 10, **dadurch gekennzeichnet, dass** die Biotransformation in einem zweiphasigen System durchgeführt, wird.

12. Verfahren nach Anspruch 11, **dadurch gekennzeichnet, dass** die Biotransformation in einem zweiphasigen System ohne Co-Solventien durchgeführt wird.

13. Verfahren nach einem der Ansprüche 1 bis 12, **dadurch gekennzeichnet, dass** die Biotransformation in einem Medium mit einer verringerten Menge M an Kohlenstoffquelle durchgeführt wird.

14. Verfahren nach Anspruch 13, **dadurch gekennzeichnet, dass** die verringerte Menge M an Kohlenstoffquelle M < 50 gL⁻¹ ist.

15. Verfahren nach einem der Ansprüche 1 bis 14, **dadurch gekennzeichnet, dass** die Reaktion in einem Rührkessel-, Oberflächen- oder Festbettreaktior durchgeführt wird.

16. Verfahren nach einem der Anspüche 1 bis 15, **dadurch gekennzeichnet, dass** als aromaaktive Terpene terpenoide Alkohole, Epoxide, Aldehyde, Ketone, Mehrfach-Alkohole, Carbonyle und Carbonylalkohole erhalten werden.

17. Verfahren nach einem der Ansprüche 1 bis 16, **dadurch gekennzeichnet, dass** Piperiton, Isopiperiton, Isopiperitenol, Isopiperitenon, Perillaaldehyd, Carvon, Carveol, Linalool, Linalooloxid, Terpineol sowie Nootkatol und Nootkaton erhalten werden.

18. Verfahren nach einem der Ansprüche 1 bis 17, **dadurch gekennzeichnet, dass** die Transformationsprodukte aus zellulären Kompartimenten oder Fraktionen isoliert werden.

19. Verfahren nach einem der Ansprüche 1 bis 18, **dadurch gekennzeichnet, dass** zuerst enantioselektiv R-(+)-Limonen zu cis-(+)-Carveol und S-(-)-Limonen zum trans-(-)-Carveol biotransformiert wird und anschließend trans-(-)-Carveol zu R-(-)-Carvon.

20. Verfahren nach Anspruch 19, **dadurch gekennzeichnet, dass** die enantioselektive Biotränsformierung von R-(+)-Limonen zu cis-(+)-Carveol mit Fusarium spec. als Biokatalysator durchgeführt wird.

21. Verfahren nach Anspruch 19, **dadurch gekennzeichnet, dass** die enantioselektive Umwandlung von trans-(-)-Carveol zu R-(-)-carvon mit Pleurotus spec. als Biokatalysator durchgeführt wird.

22. Verfahren nach einem der Ansprüche 1 bis 21, **dadurch gekennzeichnet, dass** bicyclische Sesquiterpene zu β-Nootkatol und anschließend zu Nootkaton transformiert werden.

23. Verfahren nach Anspruch 22, **dadurch gekennzeichnet, dass** die Transformieiung bicyclischer Sesquiterpene zu β-Nootkatol und anschließend zu Nootkaton mit Chaetomium spec. durchgeführt wird.

## Claims

1. A process for producing flavor-active terpenes from terpene hydrocarbons by means of a selective biotransformation and with the aid of micro-organisms of the classes of ascomycetes, basidiomycetes and deuteromycetes, **characterized in that** a lyophilized mycelium is used which is first rehydrated and then mixed with the substrate.

2. A process according to claim 1, **characterized in that** the mycelium cells are additionally permeated by means of an ultrasonic treatment and/or extrusion.

3. A process according to any one of claims 1 or 2, **characterized in that** said biotransformation is done in submerged culture.

4. A process according to any one of claims 1 to 3, **characterized in that** said biotransformation is carried out in an enantioselective, stereoselective and/or regioselective manner.

5. A process according to any one of claims 1 to 4, **characterized in that** the micro-organisms used are representatives of Fusarium, Pleurotus, Penicillium and Chaetomium.

6. A process according to any one of claims 1 to 5, **characterized in that** the micro-organisms used are Fusarium proliferatus, Pleurotus sapidus, Penicillium citrinum and Chaetomium globosum.

7. A process according to any one of claims 1 to 6, **characterized in that** the terpene hydrocarbons used are monoterpenes and sesquiterpenes.

8. A process according to any one of claims 1 to 7, **characterized in that** the terpene hydrocarbons used are limonene, pinene, valencene, farnesene, thymol and dimethylallylalcohol.

9. A process according to any one of claims 1 to 8, **characterized in that** the terpene hydrocarbons used are R-(+)-limonene or S-(-)-limonene.

10. A process according to any one of claims 1 to 9, **characterized in that** an enzyme induction is carried out in the lyophilized mycelium by adding a substrate prior to the biotransformation.

11. A process according to any one of claims 1 to 10, **characterized in that** the biotransformation is carried out in a two-phase system.

12. A process according to claim 11, **characterized in that** the biotransformation is carried out in a two-phase system without co-solvents.

13. A process according to any one of claims 1 to 12, **characterized in that** the biotransformation is carried out in a medium comprising a reduced amount M of a carbon source.

14. A process according to claim 13, **characterized in that** the reduced amount M of a carbon source is M < 50gl⁻¹.

15. A process according to any one of claims 1 to 14, **characterized in that** the reaction is carried out in a stirred-tank reactor, a surface film reactor or a fixed-bed reactor.

16. A process according to any one of claims 1 to 15, **characterized in that** the flavor-active terpenes obtained are terpenoid alcohols, epoxides, aldehydes, ketones, polyalcohols, carbonyls and carbonyl alcohols.

17. A process according to any one of claims 1 to 16, **characterized in that** piperitone, iso-piperitone, isopiperitenol, isopiperitenone, perillaaldehyde, carvone, carveol, linalool, linalool oxide, terpineol as well as nootkatol and nootkatone are obtained.

18. A process according to any one of claims 1 to 17, **characterized in that** the transformation products are isolated from cellular compartments or fractions.

19. A process according to any one of claims 1 to 18, **characterized in that**, in an enantioselective manner, first R-(+)-limonene is biotransformed into cis-(+)-carveol and S-(-)-limonene is biotransformed into trans-(-)-carveol and subsequently trans-(-)-carveol is biotransformed into R-(-)-carvone.

20. A process according to claim 19, **characterized in that** the enantioselective biotransformation of R-(+)-limonene into cis-(+)-carveol is carried out using Fusarium spec. as a biocatalyst.

21. A process according to claim 19, **characterized in that** the enantioselective transformation of trans-(-)-carveol into R-(-)-carvone is carried out using Pleurotus spec. as a biocatalyst.

22. A process according to any one of claims 1 to 21, **characterized in that** bicyclic sesquiterpenes are transformed into β-nootkatol and subsequently into nootkatone.

23. A process according to claim 22, **characterized in that** the transformation of bicyclic sesquiterpenes into β-nootkatol and subsequently into nootkatone is carried out using Chaetomium spec.

## Revendications

1. Procédé de production de terpènes à effet aromatique à partir d'hydrocarbures terpéniques au moyen d'une biotransformation sélective et à l'aide de microorganismes des classes ascomycètes, basidiomycètes et deutéromycètes, **caractérisé en ce qu'**on utilise un mycèle lyophilisé qui est d'abord réhydraté puis mélangé avec le substrat.

2. Procédé selon la revendication 1, **caractérisé en ce que** les cellules de mycèle sont traversées en plus par un traitement aux ultrasons et/ou par extrusion.

3. Procédé selon une des revendications 1 ou 2, **caractérisé en ce que** la biotransformation est effectuée en culture immergée.

4. Procédé selon une des revendications 1 à 3, **caractérisé en ce que** la biotransformation est effectuée de façon énantiosélective, stéréosélective et/ou régiosélective.

5. Procédé selon une des revendications 1 à 4, **caractérisé en ce que**, en tant que microorganismes, on utilise des représentants de fusarium, de pleurotus, de pénicillium et de chaétomium.

6. Procédé selon une des revendications 1 à 5, **caractérisé en ce que**, en tant que microorganismes, on utilise fusarium proliferatus, pleurotus sapidus, penicillium citrinum et chaetomium globosum.

7. Procédé selon une des revendications 1 à 6, **caractérisé en ce que**, comme hydrocarbures terpéniques, on utilise des monoterpènes et des sesquiterpènes.

8. Procédé selon une des revendications 1 à 7, **caractérisé en ce que**, comme hydrocarbures terpéniques, on utilise limonène, pinène, valencène, farnésène, thymol et de l'alcool diméthylallyle.

9. Procédé selon une des revendications 1 à 8, **caractérisé en ce que**, comme hydrocarbures terpéniques, on utilise R-(+)-limonène ou S-(-)-limonène.

10. Procédé selon une des revendications 1 à 9, **caractérisé en ce que**, dans le mycèle lyophilisé, on effectue, avant la biotransformation, une induction enzymatique par addition de substrat.

11. Procédé selon une des revendications 1 à 10, **caractérisé en ce que** la biotransformation est effectuée dans un système à deux phases.

12. Procédé selon la revendication 11, **caractérisé en ce que** la biotransformation est effectuée dans un système à deux phases sans co-solvant.

13. Procédé selon une des revendications 1 à 12, **caractérisé en ce que** la biotransformation est effectuée dans un milieu avec une quantité réduite M de source de carbone.

14. Procédé selon la revendication 13, **caractérisé en ce que** la quantité réduite M de source de carbone M < 50 gL⁻¹.

15. Procédé selon une des revendications 1 à 14, **caractérisé en ce que** la réaction est effectuée dans un réacteur à cuve malaxeuse, superficiel ou à lit fixe.

16. Procédé selon une des revendications 1 à 15, **caractérisé en ce que**, comme terpènes à effet aromatique, on obtient des alcools terpénoïdes, des époxides, des aldéhydes, des cétones, des polyalcools, des carbonyles et des carbonyles-alcools.

17. Procédé selon une des revendications 1 à 16, **caractérisé en ce que** l'on obtient du pipéritone, de l'isopipéritone, de l'isopipériténol, de l'isopipériténone, du périallaaldéhyde, du carvone, du carvéol, du linalol, de l'inalol oxyde, du terpinéol ainsi que du nootkatol et du nootkatone.

18. Procédé selon une des revendications 1 à 17, **caractérisé en ce que** les produits de transformation sont isolés à partir de fractions ou compartiments cellulaires.

19. Procédé selon une des revendications 1 à 18, **caractérisé en ce que** d'abord R-(+)-limonène est biotransformé de façon énantiosélective en cis-(+)-carvéol, et S-(-)-limonène est biotransformé en trans-(-)-carvéol, puis trans-(-)-carvéol est biotransformé en R-(-)-carvone.

20. Procédé selon la revendication 19, **caractérisé en ce que** que la biotransformation énantiosélective de R-(+)-limonène en cis-(+)carvéol est effectuée avec fusarium spec. en tant que biocatalyseur.

21. Procédé selon la revendication 19, **caractérisé en ce que** la conversion énantiosélective de trans-(-)-carvéol en R-(-)-carvone est effectuée avec pleutorus spec. en tant que biocatalyseur.

22. Procédé selon une des revendications 1 à 21, **caractérisé en ce que** des sesquiterpènes bicycliques sont transformés en β-nootkatol et puis en nootkatone.

23. Procédé selon la revendication 22, **caractérisé en ce que** la transformation de sesquiterpènes bicycliques en β-Nootkatol et puis en nootkatone est effectuée avec chaetomium spec.
